# EUROPEAN PATENT SPECIFICATION

(11) **EP 3 467 090 B1**
(45) Date of publication and mention of the grant of the patent: **20.08.2025**
(21) Application number: 17802258.8
(22) Date of filing: 26.05.2017
(51) Int. Cl.: C12G 1/06

(54) **SYSTEM FOR THE PROPAGATION OF YEAST AND ADAPTATION FOR SECONDARY FERMENTATION IN THE PRODUCTION OF SPARKLING WINES**
SYSTEM ZUR VERMEHRUNG VON HEFE UND ANPASSUNG FÜR EINE SEKUNDÄRE GÄRUNG BEI DER HERSTELLUNG VON SCHAUMWEIN
SYSTÈME DE MULTIPLICATION DE LEVURES ET D'APTATION POUR UNE SECONDE FERMENTATION DANS LA PRODUCTION DE VINS MOUSSEUX

(30) Priority: 27.05.2016 ES 201630695
(43) Date of publication of application: 10.04.2019
(73) Proprietor: Inbiolev, S.L., 31195 Aizoain (ES)
(72) Inventor: GARCIA YOLDI, David, 31195 Aizoain (ES)
(86) International application number: PCT/ES2017/070360
(87) International publication number: WO 2017/203090

(56) References cited:
- WO-A1-2011/058585
- DE-A1- 3 045 588
- DE-A1- 4 429 809
- ES-A6- 2 005 443
- GB-A- 953 013
- US-A- 4 948 598
- FUMI M D ET AL.: "BULK SPARKLING WINE PRODUCTION BY EXTERNAL ENCAPSULATED YEAST BIOREACTOR", BIOTECHNOLOGY LETTERS, vol. 11, no. 11, 1989, pages 821 - 824, XP055441839, ISSN: 0141-5492
- BORRULL ANNA ET AL.: "New insights into the physiological state of Saccharomyces cerevisiae during ethanol acclimation for producing sparkling wines", FOOD MICROBIOLOGY, vol. 54, April 2016 (2016-04-01), London, pages 20 - 29, XP055561871, ISSN: 0740-0020

## Description

### OBJECT OF THE INVENTION

The present invention refers to a system of propagating "pre-ferment" for the second fermentation in the production or elaboration of sparkling wines, being applicable in the tirage of cavas or champagne and in general in sparkling wines.

The invention consists of a first phase only of yeast propagation in oxidative phase without adaptation to ethanol so that in a second phase the sustained and programmed adaptation to ethanol is generated.

Both the preliminary cellular process and the adaptation of these yeasts to the ethanol take place in an automatically programmed bioreactor, in which apart from propagating the yeasts, it adapts to the ethanol, to the pH and to the temperature.

### BACKGROUND TO THE INVENTION

Traditionally, the propagation of yeasts for second fermentation in the generation of sparkling wines is done by adding wine from the beginning of the process and in an imprecise manner, thus the yeast adaptation process is not very smooth, is harmful to the physiology of the yeasts, no sustainment, producing cell damage, with low viability, resulting results yeasts which are not very healthy, logically impacting on the low quality of the sparkling wines obtained.

Traditional systems require the use of high quantity yeasts, so the application of the first propagation process described in the present report represents significant economic savings in yeasts.

Furthermore, in traditional systems, the process is carried out without any type of automatism, with no possibility of adapting precisely, in a fully controlled manner and automatically the second fermentation yeasts to the temperature, pH and ethanol.

The patent DE 30 45 588 A1 describes a procedure for the continuous flow production of sparkling wines, flow of the so-called "continuous" which, in addition to being extremely complex, presents notable and well-known disadvantages compared to "discontinuous" processes such as the one described in EP, process which in this case is non-continuous (discontinuous) and separated into 2 phases. In the process of this patent DE 30 45 588 A1 the yeast will inevitably degenerate and lose quality over time, in addition to generating possible contamination precisely because it is a "continuous" process that entails such a danger: if contamination by any micro-organism occurs, however minimal, there will be a subsequent dangerous exponential growth of that micro-organism. The patent DE 30 45 588 A1 does not provide high yield biomass, describing a multiplication of yeasts based on fermentation processes, being difficult that in presence of ethanol as described high biomass can be achieved, being the ethanol toxic for the yeasts, on the other hand when air is supplied together with the ethanol the wine oxidizes, complicating even more the result, accordingly it is not possible to generate high biomass, neither healthy nor young cells, as those achieved with the procedure described in the present invention EP. On the other hand the patent DE 30 45 588 Al does not provide for the oxygenation by introducing air or stirring by means of paddles, which is a new deficiency.

Finally the patent WO 2011 / 0585585 A1 discloses an "automated wenemaking system and winemaking method thereof". This refers to controlling winemaking processes with sensors, but without talking about sparkling wines or making a pre-fermenting (foot of vat) for sparkling wines, only controlling the winemaking process, but not considering the multiplication of yeasts or their adaptation for a second fermentation.

### DESCRIPTION OF THE INVENTION

The system which is advocated resolves in a fully satisfactory manner the problem described above in each of the aspects commented.

To do so, it is based on propagating the yeasts without adding wine, so as to obtain sequentially an adaptation of the yeasts in a manner that is smooth, sustained and programmed with the least possible cell damage, offering optimum viability and in short a better quality of resulting sparkling wines.

In addition, using the system of the invention the process is undertaken in a planned and automatic way without the oenologist having to intervene as is required traditionally, allowing gradual adaptation to the temperature and pH and of course to the ethanol.

More specifically, the system of the invention is based on the combination of a bioreactor and a mixing tank, mounted on a frame, with a common programmed pump and a control panel or system which automates the process.

With regard to the bioreactor, it is equipped with a motor with stirring blades as well as air diffusers attached to air filters, where the smallest is 0.01 microns, so sterile air enters the reactor, while the filters are attached via the appropriate piping to a compressor.

The bioreactor also has a temperature control system via cooling chambers of cooling liquid or glycol water, the entry of which is controlled by electro-valve, with resistances inside the bioreactor itself which complement the temperature control chambers.

With regard to the control panel, a frequency converter of the motor is controlled using an automaton, enabling modification of both the motor revolutions and oxygenation using a flow sensor, allowing entry of the necessary quantity of oxygen to each previously programmed phase for the yeasts, while also controlling the electro-valve and resistance for programmed temperature and pump control between the bioreactor and the mixing tank.

In accordance with these characteristics, the propagation and adaptation process of the yeasts for second fermentation in the generation of sparkling wines is as follows:
- The bioreactor is filled to half its volume, with the ideal medium for the yeast propagation without the presence of ethanol.
- The mixing tank is filled with the liquor (wine, sugar, nutritive elements and/or yeast protectors).
- The yeast is activated outside the bioreactor and once activated it is added to the bioreactor.
- The process commences with a first programme of the controller, which corresponds to yeast propagation, a process which lasts one day and in which only the bioreactor is involved. At this stage of the process, the conditions of revolutions per minute of the mixing motor are set in the corresponding programme, as well as the flow sensor conditions for the entry of filtered air and temperature conditions.
- Once the yeast propagation has passed, automatically the bioreactor enters a second programme, changing the conditions of revolutions per minute, air inlet and temperature, and activating the mixer and the pump to add to the bioreactor the quantities defined during the time the liquor is in the mixing tank. This facilitates better adaptation and greater viability of the yeasts in the adaptation to ethanol, a process that lasts one day.
- Once the second programme defined in the previous point has passed, a third programme is activated where once again the conditions of revolutions per minute of the motor, temperature and air inlet change, and liquor continues to enter the mixing tank steadily. This process lasts 1 day.
- After this third programme, the yeasts have now been propagated and adapted to the ethanol, pH and temperature, all having been done automatically, by which time the winemaker can use them for the second fermentation tirage. As the tirage is normally expected to take 5 more days, following the 3 days pre-fermenting, during these 5 days the bioreactor moves into a fourth programme of maintenance of the "pre-ferment" where once again the conditions of revolutions per minute of the motor, air inlet and temperature change. At this stage, the mixing tank has emptied and stopped just like the corresponding programmed pump.

The bioreactor described in the system serves to propagate yeasts for any wine, and it is sufficient simply to activate the first programme.

It is also designed to propagate lactic acid bacteria, based on a programme of lactic acid bacteria propagation where the conditions of revolutions per minute, temperature, oxygenation and time of the process change for 65 hours.

Finally the bioreactor is also designed to carry out autolysis or stirring of fine lee yeasts, based on a yeast autolysis programme.

### DESCRIPTION OF THE DRAWINGS

To complement the description given below and with the aim of helping towards better comprehension of the characteristics of the invention, in accordance with a preferred example of the practical implementation of the same, included as an integral part of said description is a drawing which by way of illustration but not exhaustive, represents the following:

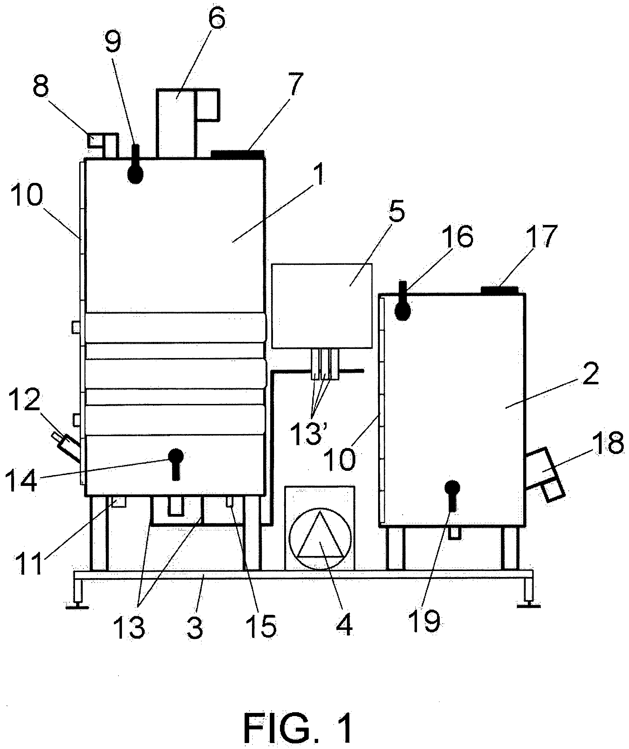

Figure 1. A schematic upright view of a system of yeast propagation and adaptation for second fermentation in the generation of sparkling wines undertaken in accordance with the aim of the present invention.

### PREFERRED EMBODIMENT OF THE INVENTION

Apparent in the drawing, it is possible to observe how the recommended device includes a bioreactor (1) in combination with a mixing tank (2), both mounted on a common frame (3) and between them a programmed pump (4), also including a control panel (5) which automates the process, and which includes a tactile screen as seen in the drawing.

The bioreactor (1) includes a motor with stirring blades (6), a folding upper door (7), gas outlet (8), a cleaning ball (9), as well as a measuring scale (10) and cooling chambers which in combination with an internal resistance (11) makes it possible to control the temperature inside said bioreactor(1).

Optionally, it includes an inlet (12) for pH meter.

It also includes an air inlet or diffusors (13) with discharge valves.

In the figure you can see how the device includes sample taking (14) consistent with the bioreactor (1) and a probe (15).

The mixing tank (2) also includes a cleaning ball (16), a folding upper door (17), a stirring motor (18) and sample taking (19) and consistent with the diffusors (13) that determine the entry of air these are also linked to the corresponding air filters (13').

According to the layout, the procedure by use of the system as per present description is as follows:
The bioreactor (1) is filled to approximately half of its volume, with the ideal medium for the propagation of yeasts.

The mixing tank (2) is filled with the liquor.

The yeast is activated outside the bioreactor (1) and once activated it is added to the bioreactor so as to start the process with a first controller programme for yeast propagation inside the bioreactor (1), establishing the conditions of r.p.m. of the stirring motor, flow sensor conditions for air inlet and conditions of temperature, so that once the propagation is obtained, the bioreactor enters a second programme changing the conditions previously described, activating the mixing tank (2) and the programmed pump (4) to add the quantities of liquor to the bioreactor (1) itself, a process that lasts 1 day.

Once programme 2 has finalised, it passes to the third programme where the conditions of revolutions, temperature and air flow change, with liquor continuing to enter steadily from the mixing tank (2) to the bioreactor (1), a programme that lasts 1 day.

The yeasts, now propagated and adapted to the ethanol, pH and temperature, having finalised the third programme, can be used for the tirage in the second fermentation, so that during the time the tirage lasts (approximately 5 days) the bioreactor (1) move into a fourth programme of maintenance where once again the conditions of revolutions, air inlet and temperature change.

## Claims

1. System of yeast propagation and adaptation for second fermentation in the generation of sparkling wines, characterised as comprising a bioreactor (1) for yeast propagation and a mixing tank (2) aimed at containing the liquor, including a programmed pump (4) between the bioreactor (1) and the mixing tank (2), as well as a programmable automaton with a control panel (5) for process automation.

2. System of yeast propagation and adaptation for second fermentation in the generation of sparkling wines, as per claim 1, characterised because the bioreactor (1) includes a motor with mixing blades (6), as well as air diffusors (13) attached to corresponding filters (13'), including also a cooling chamber coupled to internal resistances (11) of temperature control in the heart of said bioreactor (1); having been provided with a flow sensor with means of control to regulate the entry of filtered air required by the yeasts.

3. System of yeast propagation and adaptation for second fermentation in the generation of sparkling wines, as per claims 1 and 2, characterised because the bioreactor (1), mixing tank (2) and programmed pump (4) are mounted on a common frame (3).

4. System of yeast propagation and adaptation for second fermentation in the generation of sparkling wines, as per claims 1 to 3, characterised because the mixing tank (2) includes a stirring motor (18).

5. System of yeast propagation and adaptation for second fermentation in the generation of sparkling wines, as per claims 1 to 4, characterised because both the bioreactor (1) and the mixing tank (2) include corresponding folding doors (7) and (17) as well as sample taking (14) and (19).

6. System of yeast propagation and adaptation for second fermentation in the generation of sparkling wines, as per claims 1 to 5, characterised because the bioreactor (1) includes a cleaning ball (9) and a measuring scale (10).

7. System of yeast propagation and adaptation for second fermentation in the generation of sparkling wines, as per claims 1 to 5, characterised because the mixing tank (2) includes a cleaning ball (16) and a measuring ruler (10).

8. Procedure for the propagation of yeast and adaptation for secondary fermentation in the production of sparkling wines by use of the system as per claim 1, **characterized in that** it establishes a first phase only of yeast propagation in oxidative phase without adding wine and a second phase of sustained and programmed adaptation of these yeast to ethanol, both phases taking place in an automatically programmed bioreactor (1), in which apart from propagating the yeast, it adapts to the ethanol, to the pH and to the temperature.

9. Procedure for the propagation of yeast and adaptation for secondary fermentation in the production of sparkling wines, as per claim 8, characterized because once activated the yeast this is added to the bioreactor (1), establishing by a first controller programme the conditions of revolutions per minute of the stirring motor, flow sensor conditions for air inlet and conditions of temperature to yeast propagation, a process which lasts one day, so that once the propagation is obtained, the bioreactor (1) enters a second programme changing the conditions previously described, activating the mixing tank (2) and the programmed pump (4) to add the quantities of liquor to the bioreactor (1), a process that lasts one day, once program 2 has finalised, it passes to the third programme where the conditions of revolutions, temperature and air flow change, with liquor continuing to enter steadily from the mixing tank (2) to the bioreactor (1), a process that lasts one day, so that the end of the third programme the yeasts that already multiplied and adapted to the ethanol, pH and temperature are used for the tirage in the second fermentation, so that once emptied the mixing tank (2) and stopped the programmed pump (4), the bioreactor (1) move into a fourth programme of maintenance for the duration of the tirage where once again the conditions of revolutions, air inlet and temperature change.

## Patentansprüche

1. System zur Vermehrung von Hefe und Anpassung für eine sekundäre Gärung bei der Herstellung von Schaumwein, **dadurch gekennzeichnet, dass** es einen Bioreaktor (1) für die Vermehrung von Hefe und einen für die Fülldosage vorgesehenen Mischtank (2) sowie eine programmierte Pumpe (4) zwischen dem Bioreaktor (1) und dem Mischtank (2) und ein programmierbares Steuergerät mit Bedientafel (5) für die Prozessautomatisierung umfasst.

2. System zur Vermehrung von Hefe und Anpassung für eine sekundäre Gärung bei der Herstellung von Schaumwein gemäß Anspruch 1, **dadurch gekennzeichnet, dass** der Bioreaktor (1) mit einem Motor mit Rührflügeln (6) sowie mit Luftdurchlässen (13) ausgestattet ist, die mit entsprechenden Filtern (13') verbunden sind, und darüber hinaus für die Temperaturkontrolle im Innern des Bioreaktors (1) über einen Kühlmantel verfügt, der internen Widerständen (11) zugeordnet ist; außerdem ist er mit einen Durchflussregler mit Steuervorrichtung zum Regulieren der Zufuhr der von der Hefe benötigten gefilterten Luft ausgestattet.

3. System zur Vermehrung von Hefe und Anpassung für eine sekundäre Gärung bei der Herstellung von Schaumwein gemäß den Ansprüchen 1 und 2, **dadurch gekennzeichnet, dass** der Bioreaktor (1), der Mischtank (2) und die programmierte Pumpe (4) auf einen gemeinsamen Grundrahmen (3) montiert sind.

4. System zur Vermehrung von Hefe und Anpassung für eine sekundäre Gärung bei der Herstellung von Schaumwein gemäß den Ansprüchen 1 bis 3, **dadurch gekennzeichnet, dass** der Mischtank (2) einen Rührmotor (18) umfasst.

5. System zur Vermehrung von Hefe und Anpassung für eine sekundäre Gärung bei der Herstellung von Schaumwein gemäß den Ansprüchen 1 bis 4, **dadurch gekennzeichnet, dass** sowohl der Bioreaktor (1) als auch der Mischtank (2) entsprechende Klapptüren (7) und (17) Vorrichtungen für Probeentnahmen (14) und (19) umfassen.

6. System zur Vermehrung von Hefe und Anpassung für eine sekundäre Gärung bei der Herstellung von Schaumwein gemäß den Ansprüchen 1 bis 5, **dadurch gekennzeichnet, dass** der Bioreaktor (1) mit einer Reinigungskugel (9) und einem Maßstab (10) ausgestattet ist.

7. System zur Vermehrung von Hefe und Anpassung für eine sekundäre Gärung bei der Herstellung von Schaumwein gemäß den Ansprüchen 1 bis 5, **dadurch gekennzeichnet, dass** der Mischtank (2) mit einer Reinigungskugel (16) und einem Maßstab (10) ausgestattet ist.

8. Verfahren zur Vermehrung von Hefe und Anpassung für eine sekundäre Gärung bei der Herstellung von Schaumwein unter Verwendung des Systems gemäß Anspruch 1, **dadurch gekennzeichnet, dass** dabei eine erste Phase reiner Hefevermehrung in oxidativer Phase ohne Zugabe von Wein festgelegt wird, sowie eine zweite Phase anhaltender und programmierter Anpassung dieser Hefe an Ethanol, wobei beide Phasen in einem automatisch programmierten Bioreaktor (1) vollzogen werden, in dem neben der Vermehrung der Hefe auch deren Anpassung an Ethanol, pH-Wert und Temperatur erfolgen.

9. Verfahren zur Vermehrung von Hefe und Anpassung für eine sekundäre Gärung bei der Herstellung von Schaumwein gemäß Anspruch 8, **dadurch gekennzeichnet, dass** nach der Aktivierung der Hefe diese in den Bioreaktor (1) gegeben und durch ein erstes Steuerungsprogramm die Bedingungen der Umdrehungen des Rührmotors pro Minute, die Bedingungen des Durchflussreglers für den Lufteinlass und die Temperaturbedingungen für die Hefevermehrung festgelegt werden, ein Prozess, der einen Tag dauert, so dass, sobald die Vermehrung erreicht ist, der Bioreaktor (1) ein zweites Programm ausführt, mit dem die vorgenannten Bedingungen geändert und der Mischtank (2) und die programmierte Pumpe (4) aktiviert werden, um dem Bioreaktor (1) die Fülldosage-Mengen zuzuführen, ein Prozess, der einen Tag dauert; sobald das 2. Programm abgeschlossen ist, wechselt er zu einem dritten Programm, in dem die Bedingungen für Drehzahl, Temperatur und Luftstrom geändert werden, wobei von dem Mischtank (2) weiterhin kontinuierlich Fülldosage in den Bioreaktor (1) geleitet wird, ein Prozess, der einen Tag dauert, so dass nach Ablauf des dritten Programms die Hefe, die bereits vermehrt und an Ethanol, pH und Temperatur angepasst ist, in der sekundären Gärung für die Tirage verwendet wird; sobald der Mischtank (2) leer und die programmierte Pumpe (4) gestoppt ist, wechselt der Bioreaktor (1) auf ein viertes Programm, das er für die Dauer der Tirage beibehält, und in dem erneut die Bedingungen für Drehzahlen, Luftzuführung und Temperatur geändert werden.

## Revendications

1. Système de multiplication des levures et d'adaptation pour une seconde fermentation dans la production de vins mousseux, **caractérisé en ce qu'**il comprend un bioréacteur (1) destiné à la propagation des levures et une cuve de mélange (2) destinée à contenir la liqueur, comprenant une pompe programmée (4) entre le bioréacteur (1) et la cuve de mélange (2), ainsi qu'un automate programmable avec un panneau de commande (5) pour l'automatisation du procédé.

2. Système de multiplication des levures et d'adaptation pour une seconde fermentation dans la production de vins mousseux, selon la revendication 1, **caractérisé en ce que** le bioréacteur (1) comprend un moteur muni de pales de mélange (6), ainsi que des diffuseurs d'air (13) associés à des filtres correspondants (13'), comprenant également une chambre de refroidissement couplée à des résistances internes (11) pour le contrôle de la température au cœur dudit bioréacteur (1); un capteur de débit doté de moyens de contrôle a été prévu pour réguler l'entrée d'air filtré nécessaire aux levures.

3. Système de multiplication des levures et d'adaptation pour une seconde fermentation dans la production de vins mousseux, selon les revendications 1 et 2, **caractérisé en ce que** le bioréacteur (1), la cuve de mélange (2) et la pompe programmée (4) sont montés sur un châssis commun (3).

4. Système de multiplication des levures et d'adaptation pour une seconde fermentation dans la production de vins mousseux, selon les revendications 1 à 3, **caractérisé en ce que** la cuve de mélange (2) comprend un moteur d'agitation (18).

5. Système de multiplication des levures et d'adaptation pour une seconde fermentation dans la production de vins mousseux, selon les revendications 1 à 4, **caractérisé en ce que** le bioréacteur (1) et la cuve de mélange (2) comprennent respectivement des portes pliantes (7) et (17), ainsi que des dispositifs de prélèvement d'échantillons (14) et (19).

6. Système de multiplication des levures et d'adaptation pour une seconde fermentation dans la production de vins mousseux, selon les revendications 1 à 5, **caractérisé en ce que** le bioréacteur (1) comprend une boule de nettoyage (9) et une échelle de mesure (10).

7. Système de multiplication des levures et d'adaptation pour une seconde fermentation dans la production de vins mousseux, selon les revendications 1 à 5, **caractérisé en ce que** la cuve de mélange (2) comprend une boule de nettoyage (16) et une règle de mesure (10).

8. Procedure de multiplication des levures et d'adaptation pour une seconde fermentation dans la production de vins mousseux au moyen du système selon la revendication 1, **caractérisé en ce qu'**il prévoit une première phase de propagation des levures en phase oxydative sans ajout de vin, et une seconde phase d'adaptation soutenue et programmée de ces levures à l'éthanol, les deux phases se déroulant dans un bioréacteur (1) programmé automatiquement, dans lequel, en plus de la propagation des levures, celles-ci s'adaptent à l'éthanol, au pH et à la température.

9. Procedure de propagation des levures et d'adaptation pour une seconde fermentation dans la production de vins mousseux, selon la revendication 8, **caractérisé en ce qu'**une fois les levures activées, elles sont ajoutées au bioréacteur (1), un premier programme de commande établissant les conditions de révolutions par minute du moteur d'agitation, les conditions du capteur de débit pour l'entrée d'air et les conditions de température pour la propagation des levures, ce processus durant une journée ; une fois la propagation obtenue, le bioréacteur (1) passe à un second programme modifiant les conditions précédemment décrites, activant la cuve de mélange (2) et la pompe programmée (4) pour ajouter les quantités de liqueur au bioréacteur (1), ce processus durant une journée ; une fois le deuxième programme terminé, le troisième programme est lancé, modifiant les conditions de révolutions, de température et de flux d'air, la liqueur continuant d'entrer régulièrement de la cuve de mélange (2) vers le bioréacteur (1), ce processus durant une journée ; à la fin du troisième programme, les levures déjà multipliées et adaptées à l'éthanol, au pH et à la température sont utilisées pour le tirage lors de la seconde fermentation ; une fois la cuve de mélange (2) vidée et la pompe programmée (4) arrêtée, le bioréacteur (1) passe à un quatrième programme de maintien pendant la durée du tirage, où, à nouveau, les conditions de révolutions, d'entrée d'air et de température changent.
